(19) **Europäisches Patentamt / European Patent Office / Office européen des brevets**

(11) **EP 3 369 365 A1**

(12) # EUROPEAN PATENT APPLICATION
published in accordance with Art. 153(4) EPC

(43) Date of publication:
**05.09.2018 Bulletin 2018/36**

(21) Application number: **16880319.5**

(22) Date of filing: **07.03.2016**

(51) Int Cl.:
***A61B 3/103*** *(2006.01)*

(86) International application number:
**PCT/CN2016/075792**

(87) International publication number:
**WO 2017/113510 (06.07.2017 Gazette 2017/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.12.2015 CN 201511025379**

(71) Applicants:
• **Shenzhen Moptim Imaging Technique Co., Ltd.**
  **Shenzhen, Guangdong 518112 (CN)**
• **Shenzhen Certainn Technology Co., Ltd.**
  **Shenzhen, Guangdong 518112 (CN)**

(72) Inventors:
• **GUO, Shuguang**
  **Shenzhen**
  **Guangdong 518112 (CN)**
• **WAN, Mingming**
  **Shenzhen**
  **Guangdong 518112 (CN)**
• **WU, Lei**
  **Shenzhen**
  **Guangdong 518112 (CN)**
• **HE, Weihong**
  **Shenzhen**
  **Guangdong 518112 (CN)**

(74) Representative: **Loo, Chi Ching et al**
  **Albright IP Limited**
  **County House**
  **Bayshill Road**
  **Cheltenham, Gloucestershire GL50 3BA (GB)**

(54) **BINOCULAR OPTOMETRY DEVICE AND OPTOMETRY METHOD**

(57)     A binocular refractometer is provided. The binocular refractometer includes a light source, a converging lens group, a light splitting apparatus, and an optical element adjusting apparatus. The light splitting apparatus splits measuring light into two beams of light, wherein a first beam of light of the two beams of light passes through a first fundus oculi imaging light path and forms, on a first imaging unit, a first dot pattern responsive to light reflected from the fundus oculi of a first human eye, and a second beam of light of the two beams of light passes through a second fundus oculi imaging light path and forms, on a second imaging unit, a second dot pattern responsive to light reflected from the fundus oculi of a second human eye. The optical element adjusting apparatus is configured to adjust the first and second fundus oculi imaging light paths along the direction of the pupillary distance. The optical element adjusting apparatus is configured to adjust the light source along the direction of the optical axis of the measuring light to fog the first human eye and the second human eye, so that for subjects with different diopters, the first dot pattern responsive to the fundus oculi of the first human eye is clearly formed on the first imaging unit and the second dot pattern responsive to the fundus oculi of the second human eye is clearly formed on the second imaging unit. The light source is conjugated to the fundus oculi of the first human eye predetermined by the converging lens group. The light source is conjugated to the fundus oculi of the second human eye predetermined by the converging lens group. The binocular refractometer can measure binocular diopters and the pupillary distance, and can accurately determine binocular astigmatism and binocular astigmatic axis angle.

EP 3 369 365 A1

FIG.1

**Description**

TECHNICAL FIELD

The present disclosure relates to the field of optical measurement technology, and particularly to a binocular refractometer and a method thereof.

BACKGROUND

In the related art, although devices for measuring ocular visual acuity using geometrical optics methods can measure two eyes at the same time, usually only one parameter can be measured. For instance, in US patent (Patent No. 5777718), only refractive power is measured. Chinese patent (Publication No. CN101718542B), named optical ranging device and portable refractometer thereof, provides a method and a device for measuring diopters of human eyes by measuring working distance between the human eyes and an instrument. However, only refractive power is measured.

More parameters can be measured using wavefront optics methods, but single-eye measurements are usually only possible with this method. Because wavefront measurements are sensitive to light and have high demands, it is difficult to use spectroscopic components.

International patent application (Publication NO. WO2005048829A2) provides an apparatus for measuring eyesight by using a wavefront method, which can measure the two eyes simultaneously. However, it adopts complicated optical, mechanical, electrical facilities and computer software, and therefore the equipment is complicated and the cost is high. In addition, measuring accuracy of high refractive error is not high.

Chinese patent CN102988022A, named ocular error measurement device and method, provides an ocular error measurement device and method using the wavefront optics method, but only single-eye and the refractive power of the human eye can be measured.

Therefore, it is desirable to propose a more simple and efficient eye disease measuring device that can measure a variety of eye disease parameters (such as, diopters of two eyes, astigmatism, astigmatic axis angle, etc.) and the pupillary distance and solve the problem that accuracy of the high refractive error measured using wavefront aberration measurement diopter method is not high.

SUMMARY

The disclosure aims to solve the problem that accuracy of the high refractive error measured using wavefront aberration measurement diopter method is not high by measuring diopters of two eyes through wavefront aberration measurement principle and can measure the diopters, astigmatism, astigmatic axis angle, and the pupillary distance.

According to the disclosure, there is provided a binocular refractometer. The binocular refractometer includes a light source configured to provide measuring light and a third lens group configured to converge the measuring light provided by the light source. The binocular refractometer further includes a light splitting apparatus. The light splitting apparatus is configured to split the measuring light provided by the light source and converged by the third lens group into two beams of light. A first beam of light of the two beams of light passes through a first fundus oculi imaging light path and illuminates a first human eye, and the returned first beam of light carrying fundus oculi information of the first human eye forms, on a first imaging unit in the first fundus oculi imaging light path, a first dot pattern responsive to light reflected from the fundus oculi of the first human eye. A second beam of light of the two beams of light passes through a second fundus oculi imaging light path and illuminates a second human eye, and the returned second beam of light carrying fundus oculi information of the second human eye forms, on a second imaging unit in the second fundus oculi imaging light path, a second dot pattern responsive to light reflected from the fundus oculi of the second human eye. The binocular refractometer further includes an optical element adjusting apparatus. The optical element adjusting apparatus is configured to adjust optical elements in the first fundus oculi imaging light path and optical elements in the second fundus oculi imaging light path along the direction of the pupillary distance as well as adjust the light source along the direction of the optical axis of the measuring light to fog the first human eye and the second human eye, whereby, for subjects with different diopters, the first dot pattern relating to the fundus oculi of the first human eye is clearly formed on the first imaging unit and the second dot pattern relating to the fundus oculi of the second human eye is clearly formed on the second imaging unit. The light source is conjugated to the fundus oculi of the first human eye predetermined by the third lens group. The light source is conjugated to the fundus oculi of the second human eye predetermined by the third lens group.

As one implementation, the first fundus oculi imaging light path includes a first beam divider, a first lens group, a first micro-lens array, and the first imaging unit sequentially arranged along the first fundus oculi imaging light path. The second fundus oculi imaging light path includes a first reflector, a second beam divider, a second lens group, a second micro-lens array, and the second imaging unit sequentially arranged along the second fundus oculi imaging light path. The light splitting apparatus is a third beam divider. The first dot pattern is formed on the first imaging unit, by converging the first beam of light carrying the fundus oculi information of the first human eye through the first micro-lens array. The second dot pattern is formed on the second imaging unit, by converging the second beam of light carrying the fundus oculi information of the second human eye through the second micro-lens array.

As one implementation, the first fundus oculi imaging light path further includes a second reflector and a third reflector that are disposed between the first beam divider and the first lens group. The second fundus oculi imaging light path further includes a fourth reflector and a fifth reflector that are disposed between the second beam divider and the second lens group.

As one implementation, the first fundus oculi imaging light path further includes a first Dove prism disposed between the first human eye and the first beam divider. The second fundus oculi imaging light path further includes a second Dove prism disposed between the second human eye and the second beam divider. The first Dove prism and the second Dove prism have the same specifications.

As one implementation, the first fundus oculi imaging light path further includes a first prism disposed between the first human eye and the first beam divider. The second fundus oculi imaging light path further includes a second prism disposed between the second human eye and the second beam divider. The first prism and the second prism have the same specifications.

As one implementation, the first prism has slopes (UVWX and U1V1W1X1) both forming an angle of 45 degrees with a horizontal plane, and the second prism has slopes (UVWX and U1V1W1X1) both forming an angle of 45 degrees with the horizontal plane.

As one implementation, the first fundus oculi imaging light path includes a third Dove prism, the first lens group, the first micro-lens array, and the first imaging unit sequentially arranged along the first fundus oculi imaging light path. The second fundus oculi imaging light path includes the first reflector, a fourth Dove prism, the second lens group, the second micro-lens array, and the second imaging unit sequentially arranged along the second fundus oculi imaging light path. The third Dove prism and the fourth Dove prism have the same specifications. The light splitting apparatus is a third beam divider. The first dot pattern is formed on the first imaging unit, by converging the first beam of light carrying the fundus oculi information of the first human eye through the first micro-lens array. The second dot pattern is formed on the second imaging unit, by converging the second beam of light carrying the fundus oculi information of the second human eye through the second micro-lens array.

As one implementation, the third Dove prism has an incident surface (AD) that is a transflective surface and an emergence surface (FG) that is a totally transparent surface. An angle between the emergence surface (FG) and a reflective surface (AF) is 45 degrees, and the third Dove prism has a section (DGFA) that is an isosceles trapezoid. The fourth Dove prism has an incident surface (HL) that is a transflective surface and an emergence surface (IG) that is a totally transparent surface. An angle between the incident surface (HL) and a reflective surface (LG) is 45 degrees, and the fourth Dove prism has a section (HIGL) that is an isosceles trapezoid.

As one implementation, the first imaging unit and the second imaging unit are both complementary metal oxide semiconductor (CMOS) cameras or charge coupled device (CCD) imaging units.

According to the disclosure, there is also provided a method for measuring astigmatism and astigmatic axis angle of two eyes by using the binocular refractometer. The method includes the following operations.

At S1, measuring light is split into two beams of light by a third beam divider after the measuring light provided by a light source is converged by a third lens group. A first beam of light of the two beams of light passes through a first fundus oculi imaging light path and illuminates a first human eye, and the returned first beam of light carrying fundus oculi information of the first human eye forms, on a first imaging unit in the first fundus oculi imaging light path, a first dot pattern responsive to light reflected from the fundus oculi (Er) of the first human eye. A second beam of light of the two beams of light passes through a second fundus oculi imaging light path and illuminates a second human eye, and the returned second beam of light carrying fundus oculi information of the second human eye forms, on a second imaging unit in the second fundus oculi imaging light path, a second dot pattern responsive to light reflected from the fundus oculi (Er) of the second human eye.

At S2, the first fundus oculi imaging light path and the second fundus oculi imaging light path are adjusted by an optical element adjusting apparatus in the direction of the pupillary distance. The light source is adjusted along the direction of the optical axis of the measuring light to fog the first human eye and the second human eye, so that for subjects with different diopters, the first dot pattern relating to the fundus oculi of the first human eye is clearly formed on the first imaging unit and the second dot pattern relating to the fundus oculi of the second human eye is clearly formed on the second imaging unit.

At S3, it indicates that the two eyes of the subject have no astigmatism and astigmatic axis angle based on a determination that figures formed by four dots adjacent in horizontal and vertical directions in each of the first dot pattern displayed on the first imaging unit and the second dot pattern displayed on the second imaging unit are all squares. Conversely, it means that the two eyes of the subject have the astigmatism and the astigmatic axis angle based on a determination that figures formed by four dots adjacent in the horizontal and vertical directions in each of the first dot pattern displayed on the first imaging unit and the second dot pattern displayed on the second imaging unit are all rectangles or rhomboids.

According to the disclosure, there is also provided a method for measuring diopters of two eyes by using the binocular refractometer. The method includes the following operations.

At S1, measuring light is split into two beams of light by a third beam divider after the measuring light provided by a light source is converged by a third lens group. A first beam of light of the two beams of light passes through a first fundus oculi imaging light path and illuminates a first human eye, and the returned first beam of light carrying fundus oculi information of the first human eye forms, on a first imaging unit in the first fundus oculi imaging light path, a first dot pattern responsive to light reflected from the fundus oculi (Er) of the first human eye. A second beam of light of the two beams of light passes through a second fundus oculi imaging light path and illuminates a second human eye, and the returned second beam of light carrying fundus oculi information of the second human eye forms, on a second imaging unit in the second fundus oculi imaging light path, a second dot pattern responsive to light reflected from the fundus oculi (Er) of the second human eye.

At S2, the first fundus oculi imaging light path and the second fundus oculi imaging light path are adjusted by an optical element adjusting apparatus in the direction of the pupillary distance. The light source is adjusted along the direction of the optical axis of the measuring light to fog the first human eye and the second human eye, so that for subjects with different diopters, the first dot pattern relating to the fundus oculi of the first human eye is clearly formed on the first imaging unit and the second dot pattern relating to the fundus oculi of the second human eye is clearly formed on the second imaging unit.

At S3, a diopter (D) of the first human eye is calculated according to a distance between two adjacent dots in the first dot pattern on the first imaging unit and parameters set by the binocular refractometer. A diopter (D) of the second human eye is calculated according to a distance between two adjacent dots in the second dot pattern on the second imaging unit and the parameters set by the binocular refractometer.

According to the disclosure, it is also provided a method for measuring the pupillary distance by using the binocular refractometer. The method includes the following operations.

At S1, diopters D of two eyes of a subject are measured according to the above method for measuring diopters.

At S2, a distance K1 at which an image (Er1) deviates from a center O1 of the first imaging unit is measured, where the image (Er1) is formed on a first imaging unit by light reflected from the fundus oculi (Er) of a first human eye. A distance K2 at which an image (Er2) deviates from a center O2 of the second imaging unit is measured, where the image (Er2) is formed on a second imaging unit by light reflected from the fundus oculi (Er) of a second human eye. The pupillary distance of an adjusted system is also measured.

At S3, K1 and K2 measured at S2 are substituted for K in a formula respectively. A distance K1' at which the axis of the first human eye deviates from the optical axis of a first lens group in a first fundus oculi imaging light path and a distance K2' at which the axis of the second human eye deviates from the optical axis of a second lens group in a second fundus oculi imaging light path are calculated.

$$K' = \frac{K\left(\dfrac{m \cdot f \cdot D + 1000 f}{(m-f) \cdot D + 1000} - \dfrac{m \cdot f}{m+f}\right)}{\left(\dfrac{m \cdot f \cdot D + 1000 f}{(m-f)D + 1000} - d\right)} * \frac{m+f}{f}$$

In the formula, d is a distance, in a measuring light path, between a rear principal plane of the first lens group and the first imaging unit; f is a focal length of the first lens group in the measuring light path; D is the measured diopter of the measured eye of the subject; and m is a distance between a pupil and a front principal plane of the first lens group.

At S4, L2 obtained at S2 and K1' as well as K2' obtained at S3 are substituted into a formula L1'= L2+K1'+K2', to obtain the pupillary distance (L1') of the subject.

The advantageous effects of the disclosure are as follows.

1. According to the binocular refractometer of the disclosure, the first fundus oculi imaging light path and the second fundus oculi imaging light path are formed through splitting the measuring light into two beams of light by the light splitting apparatus. The first fundus oculi imaging light path and the second fundus oculi imaging light path are adjusted in the direction of the pupillary distance by the optical element adjusting apparatus. The light source is adjusted in the direction of the optical axis of the measuring light to fog the first human eye and the second human eye, so that for the subjects with different diopters, the first dot pattern relating to the fundus oculi of the first human eye is clearly formed on the first imaging unit and the second dot pattern relating to the fundus oculi of the second human eye is clearly formed on the second imaging unit. The binocular refractometer can measure the diopters and the pupillary distance by keeping the light source and the fundus oculi (Er) of the first human eye conjugated predetermined by the converging lens group as well as the light resource and the fundus oculi (Er) of the second human eye conjugated predetermined by the converging lens group.

2. According to the method for measuring the diopters of two eyes by using the binocular refractometer, the diopter (D) of the first human eye is calculated according to the distance between two adjacent dots in the first dot pattern on the first imaging unit (7) and the parameters set by the binocular refractometer. Likewise, the diopter (D) of the second human eye is calculated according to the distance between two adjacent dots in the second dot pattern on the second imaging unit (14) and the parameters set by the binocular refractometer. As the light source can be adjusted in the direction of the optical axis of the measuring light to fog the first human eye and the second human eye, the first dot pattern (image formed on the fundus oculi of the first human eye) relating to the fundus oculi of the first human eye can be clearly formed on the first imaging unit and the second dot pattern (image formed on the fundus oculi of the second human eye) relating to the fundus oculi of the second human eye can be clearly formed on the second imaging unit. Consequently, the measured diopters can be more precious.

3. According to the method for measuring the astigmatism and the astigmatic axis angle of two eyes by using the binocular refractometer, whether two eyes of the subject have the astigmatism and the astigmatic axis angle is determined according to whether the figures formed by four dots adjacent in the horizontal and vertical directions in each of the first dot pattern displayed on the first imaging unit and the second dot pattern displayed on the second imaging unit are all squares. Since the light source can be adjusted in the direction of the optical axis of the measuring light to fog the first human eye and the second human eye, the first dot pattern (image formed on the fundus oculi of the first human eye) relating to the fundus oculi of the first human eye can be clearly formed on the first imaging unit and the second dot pattern (image formed on the fundus oculi of the second human eye) relating to the fundus oculi of the second human eye can be clearly formed on the second imaging unit. As a result, the astigmatism and the astigmatic axis angle of the subject can be determined more accurately.

4. According to the method for measuring the pupillary distance by using the binocular refractometer, as the light source can be adjusted in the direction of the optical axis of the measuring light to fog the first human eye and the second human eye, the system pupillary distance (L2) can be obtained by adjusting the corresponding optical elements, and therefore the pupillary distance can be calculated more accurately.

BRIEF DESCRIPTION OF THE DRAWINGS

FIG. 1 illustrates an optical path diagram according to the first embodiment of the present disclosure.
FIG. 2 illustrates dot patterns responsive to light reflected from fundi oculi of two eyes of the present disclosure.
FIG. 2-1 illustrates a dot-pattern image on a first imaging unit and a dot-pattern image on a second imaging unit of the present disclosure and FIG. 2-2 illustrates a dot-pattern image on the first imaging unit and a dot-pattern image on the second imaging unit of the present disclosure.
FIG. 3 illustrates an optical path diagram according to the second embodiment of the present disclosure.
FIG. 4 illustrates an optical path diagram according to the third embodiment of the present disclosure.
FIG. 5 illustrates an optical path diagram when one Dove prism of two Dove prisms translates upward by a distance p and the other one Dove prism of the two Dove prisms translates downward by a distance p at the same time according to the third embodiment of the present disclosure.
FIG. 6 illustrates an optical path diagram according to the fourth embodiment of the present disclosure.
FIG. 7 illustrates a partial optical path diagram after a Dove prism moves upward obliquely according to the fourth embodiment of the present disclosure.
FIG. 8 illustrates an optical path diagram according to a fifth embodiment of the present disclosure.
FIG. 9 is a schematic diagram illustrating a first prism and a second prism according to the fifth embodiment of the present disclosure.
FIG. 10 illustrates a side view of the first prism and the second prism in FIG. 9 when viewed from the direction of the arrow K shown in FIG. 9.
FIG. 11 illustrates a schematic diagram when a first prism and a second prism rotate outward by $\alpha$ degrees respectively according to the fifth embodiment of the present disclosure.

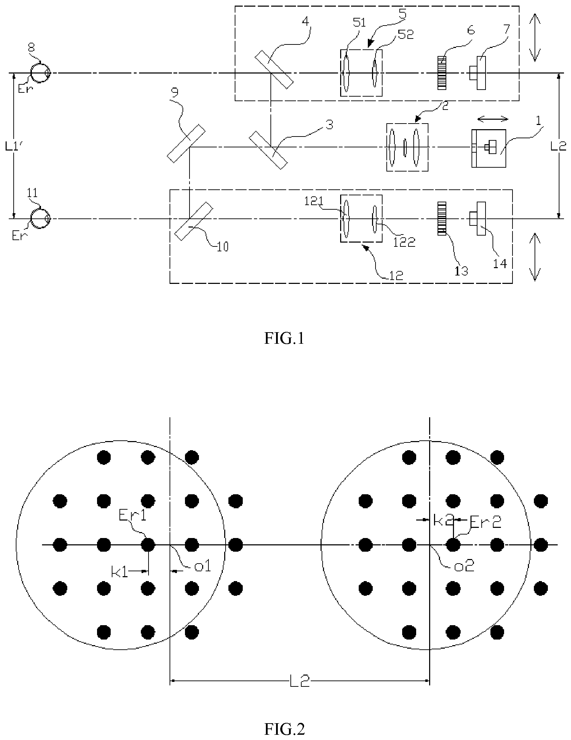

The following describes referral numbers in the figures. 1: light source; 2: third lens group; 3: third beam divider; 4: first beam divider; 5: first lens group; 6: first micro-lens array; 7: first imaging unit; 8: first human eye; 9: first reflector; 10: second beam divider; 11: second human eye; 12: second lens group; 13: second micro-lens array; 14: second imaging unit; 15: second reflector; 16: third reflector; 17: fourth reflector; 18: fifth reflector; 21: first Dove prism; 22:

second Dove prism; 23: third Dove prism; 24: fourth Dove prism; 41: first prism; 42: second prism; Er represents the fundus oculi of the first human eye 8 or the fundus oculi of the second human eye 11; Er1 represents an image formed on the first imaging unit 7 by light reflected from the fundus oculi of the first human eye 8; Er2 represents an image formed on the second imaging unit 14 by light reflected from the fundus oculi of the second human eye 11; O1 represents a center of the first imaging unit 7; O2 represents a center of the second imaging unit 14; K1 represents a distance at which an image Er1 formed on the first imaging unit 7 by the light reflected from the fundus oculi Er of the first human eye 8 deviates from the center O1 of the first imaging unit 7; K2 represents a distance at which an image Er2 formed on the second imaging unit 14 by the light reflected from the fundus oculi Er of the second human eye 11 deviates from the center O2 of the second imaging unit 14; L2 represents a system pupillary distance; L1' represents the pupillary distance ; A1, A2, A3, A4 are dots formed by the reflected light from the fundus oculi Er of the first human eye 8 on the first imaging unit 7; B1, B2, B3, B4 are dots formed by the reflected light from the fundus oculi Er of the second human eye 11 on the second imaging unit 14; a represents an upward adjustment distance of the second human eye 11; p represents a translation distance of the fourth Dove prism; $\alpha$ represents an outward rotation angle of the second prism; Q represents a center point of slope UVWX of the first prism; Q1 represents a center point of slope U1V1W1X1 of the first prism; R represents a center point of slope UVWX of the second prism; R1 represents a center point of slope U1V1W1X1 of the second prism; h represents a distance between Q and Q1 or a distance between R and R1; 31 represents an optical axis; 32 represents an optical axis; 33 represents an optical axis.

DETAILED DESCRIPTION OF ILLUSTRATED EMBODIMENTS

The present disclosure will be further described in detail hereinafter with reference to the specific embodiments and the accompanying drawings. It should be emphasized that, the following description is merely exemplary and is not intended to limit the scope of the present disclosure and its application.

First embodiment

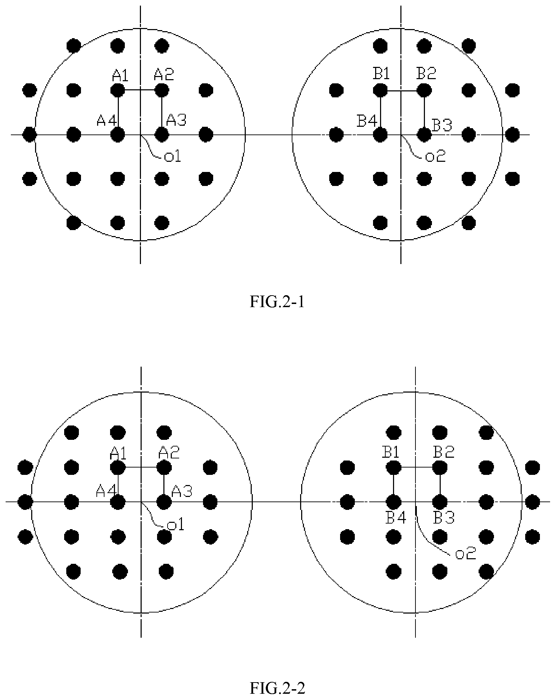

As illustrated in FIG. 1, a beam of light provided by a light source 1 passes through a third lens group 2 composed of several imaging lenses and then is incident to a third beam divider 3. A part of the beam of light is reflected by the third beam divider 3 to form reflected light, and another part of the beam of light is transmitted by the third beam divider 3 to form transmitted light. The reflected light formed is reflected by a first beam divider 4 to the fundus oculi Er of a first human eye 8 (e.g., the upper eye), and scattered by the fundus oculi Er of the first human eye 8. Reflected light carrying fundus oculi information of the first human eye 8 is returned to the first beam divider 4 and then transmitted by the first beam divider 4 to a first lens group 5, finally imaged on a first imaging unit 7 after passing through a first micro-lens array 6 to obtain a first dot pattern responsive to light reflected from the fundus oculi Er of the first human eye 8 (see Er1 illustrated in FIG. 2). The transmitted light transmitted by the third beam divider 3 is incident to a first reflector 9 and then reflected by the first reflector 9 to a second beam divider 10, reflected by the second beam divider 10 to the fundus oculi Er of a second human eye 11 (e.g., the lower eye), and scattered by the fundus oculi Er of the second human eye 11. Reflected light carrying fundus oculi information of the second human eye 11 is returned to the second beam divider 10 and then transmitted by the second beam divider 10 to a second lens group 12, finally imaged on a second imaging unit 14 after passing through a second micro-lens array 13 to obtain a second dot pattern responsive to light reflected from the fundus oculi Er of the second human eye 11 (see Er2 illustrated in FIG. 2).

A diopter is calculated according to a distance between two adjacent dots (for example, the distance between a dot A1 and a dot A2) in one of the two dot patterns in FIG. 2 as well as parameters set by this binocular refractometer. The parameters of this binocular refractometer may include focal lengths of all lenses or mirrors included in the third lens group 2, the first lens group 5, and the second lens group 12 of the embodiments, as well as the distance involved between any two of all the lenses, all the reflectors, and all the beam dividers. Therefore, diopters of two eyes of a subject are calculated according to the distances and the parameters above.

Referring to FIG. 2-1, for a subject without astigmatism (also called a cylinder) and astigmatic axis angle (also called cylinder astigmatic axis angle) in two eyes, the distance between any two adjacent dots in a dot pattern on the first imaging unit 7 are equal and the distance between any two adjacent dots in a dot pattern on the second imaging unit 14 (See FIG. 2-1) are equal, that is, a figure formed by four dots A1, A2, A3, and A4 is a square (the dots are formed by converging, on the first imaging unit 7, a beam of light carrying the fundus oculi information of the first human eye 8 through the first micro-lens array 6), and a figure formed by the four dots B1, B2, B3, and B4 is a square (the dots are formed by converging, on the second imaging unit 14, a beam of light carrying the fundus oculi information of the second human eye 11 through the second micro-lens array 13). For a subject with astigmatism and astigmatic axis angle in two eyes, a dot-pattern image displayed on the first imaging unit 7 and a dot-pattern image displayed on the second imaging unit 14 are illustrated in FIG. 2-2, that is, figures formed by any adjacent four dots in horizontal and vertical directions in FIG. 2-2 are rectangles. Assume that the four dots A1, A2, A3, and A4 in left dot pattern in FIG. 2-2 form a rectangle A1A2A3A4, it indicates that the first human eye 8 has astigmatism and astigmatic axis angle. Assume that the four dots B1, B2, B3, and B4 in right dot pattern in FIG. 2-2 form a rectangle B1B2B3B4, it indicates that the second human eye 11 has astigmatism and astigmatic axis angle. In addition, for the subject with astigmatism and astigmatic axis angle in two eyes, a parallelogram dot pattern may be displayed on the first imaging unit 7 and a parallelogram dot pattern may be displayed on the second imaging unit 14, such as a parallelogram A1A2A3A4 and a parallelogram B1B2B3B4.

Therefore, the diopters of two eyes of the subject can be measured by using this binocular refractometer and whether each of two eyes of the subject have astigmatism and astigmatic axis angle is determined by judging whether figures formed by four dots adjacent in horizontal and vertical directions in the first dot pattern and in the second dot pattern are all squares.

At the same time, this binocular refractometer can also measure the pupillary distance L1' of the subject.

In general, an initial value of a system pupillary distance L2 of this binocular refractometer is 65 mm. However, subjects of different ages and genders have different pupillary distances. Therefore, when measuring the pupillary distances of the subjects of different ages and genders, the initial value of the system pupillary distance L2 used can be 65 mm or other values. In order to obtain different system pupillary distances L2 suitable for different subjects, the first beam divider 4 and the second beam divider 10 illustrated in FIG. 1 need to be adjusted along the direction of the pupillary distance while adjusting the light source 1 along the direction of the optical axis of the measuring light, until the light reflected from the fundus oculi Er of the first human eye 8 forms a clear image on the first imaging unit 7 and the light reflected from the fundus oculi Er of the second human eye 11 forms a clear image on the second imaging unit 14 (see FIG. 2). Since the first imaging unit 7, the first micro-lens array 6, the first lens group 5, and the first beam divider 4 are on the same optical axis, when the first imaging unit 7 is adjusted, the first imaging unit 7, the first micro-lens array 6, the first lens group 5, and the first beam divider 4 need to be adjusted as a whole (see FIG. 1) until the light reflected from the fundus oculi Er of the first human eye 8 forms the clear image on the first imaging unit 7 (see FIG. 2); similarly, since the second beam divider 10, the second lens group 12, the second micro-lens array 13, and the second imaging unit 14 are on the same optical axis, when the second beam divider 10 is adjusted, the second half-mirror 10, the second lens group 12, the second micro-lens array 13, and the second imaging unit 14 need to be adjusted as a whole (see FIG. 1) until the light reflected from the fundus oculi Er of the second human eye 11 forms the clear image on the second imaging unit 14 (see FIG. 2). Therefore, the system pupillary distance L2 may be greater or less than 65 mm.

The pupillary distance L1' is calculated after adjusting the system pupillary distance the L2. The pupillary distance L1' is calculated as follows. Assume that a distance at which an image Er1 (see FIG. 2) deviates from a center O1 of the first imaging unit 7 is defined as K1, where the image Er1 is formed on the first imaging unit 7 by the light reflected from the fundus oculi Er of the first human eye 8 (see FIG. 1), and a distance at which an image Er2 (see FIG. 2) deviates from a center O2 of the second imaging unit 14 is defined as K2, where the image Er2 is formed on the second imaging unit 14 by the light reflected from the fundus oculi Er of the second human eye 11 (see FIG. 1).

$$K' = \frac{K\left(\dfrac{m \cdot f \cdot D + 1000f}{(m-f) \cdot D + 1000} - \dfrac{m \cdot f}{m+f}\right)}{\left(\dfrac{m \cdot f \cdot D + 1000f}{(m-f)D + 1000} - d\right)} * \frac{m+f}{f} \tag{1}$$

In Formula (1), d is a distance, in a measuring light path, between a rear principal plane of the first lens group 5 and the first imaging unit 7; f is a focal length of the first lens group 5 in the measuring light path; D is a diopter of one of the two measured eyes of the subject; and m is a distance between a pupil and a front principal plane of the first lens group 5. Based on the above parameters, an actual distance K1' at which an axis of the first human eye 8 deviates from an optical axis (i.e., the center of the first imaging unit 7) and an actual distance K2' at which an axis of the second human eye 11 deviates from an optical axis (i.e., the center of the second imaging unit 14) can be obtained. That is to say, by substituting K1 for K in formula (1), the actual distance K1' at which the axis of the first human eye 8 (not shown) deviates from an optical axis O1 (i.e., the center of the first imaging unit 7) can be calculated, where K1 is measured by this binocular refractometer and is a known value. By substituting K2 for K in formula (1), the actual distance K2' at which the axis of the second human eye 11 (not shown) deviates from an optical axis O2 (i.e., the center of the second imaging unit 14) can also be calculated, where K2 is measured by this binocular refractometer and is a known value. In fact, the second lens group 12 and the first lens group 5 are completely the same, the second micro-lens array 13 and the first micro-lens array 6 are completely the same, the first imaging unit 7 and the second imaging unit 14 are completely the same, a distance from a rear principal plane of the second lens group 12 to the second imaging unit 14 is also d. Consequently, when calculating K2', compared with calculating K1', other parameters are all the same except that K2 is different from K1.

After K1' and K2' are calculated, the pupillary distance L1' can be calculated according to the formula L1'= L2+K1'+K2'.

K1' can be positive, negative, or zero. Similarly, K2' can be positive, negative, or zero. When K1' is zero, it means that the light reflected from the fundus oculi Er of the first human eye 8 just passes through the center O1 of the first imaging unit 7. When K2' is zero, it means that the light reflected from the fundus oculi Er of the second human eye 11 just passes through the center O2 of the second imaging unit 14.

Since the light source 1 can be adjusted along the direction of the optical axis of the measuring light to fog the first human eye and the second human eye, for subjects with different diopters, the first dot pattern relating to the fundus oculi Er of the first human eye 8 can be always clearly formed on the first imaging unit 7, the second dot pattern relating to the fundus oculi Er of the second human eye 11 can be always clearly formed on the second imaging unit 14 (see FIG. 2).

It should be noted that, as illustrated in FIG. 1, the first lens group 5 includes two lenses and the second lens group 12 includes two lenses as well, but these are merely exemplary. The third lens group 2 includes three lenses, which is also merely exemplary. In fact, the first lens group 5 and the second lens group 12 may include only one lens respectively, for example, the first lens group 5 only includes a lens 51 and the second lens group 12 only includes a lens 121, and the definition of d remains unchanged. When the first lens group 5 and the second lens group 12 both include three or more lenses, d is a distance between a rear principal plane of the first lens group 5 after all the lenses of the first lens group 5 are combined and the first imaging unit 7.

It should be noted that, when measuring the pupillary distance and optometry, the light source 1 is conjugated to the fundus oculi Er of the first human eye 8 predetermined by the third lens group 2, and the light source 1 is conjugated to the fundus oculi Er of the second human eye 11 predetermined by the third lens group 2. That is, when a subject is in a state of ametropia, a clear image is formed on the fundus oculi Er of the first human eye 8 and a clear image is formed on the fundus oculi Er of the second human eye 11 after adjusting the light source 1 in horizontal direction; when a subject is in a state of emmetropia, the light source 1 and the fundus oculi Er of the first human eye 8 need to satisfy a conjugate condition, the light source 1 and the fundus oculi Er of the second human eye 11 need to satisfy the conjugate condition as well, in this situation, the light source 1 can realize the conjugate condition without adjustment.

The first imaging unit 7 and the second imaging unit 14 are both complementary metal oxide semiconductor (CMOS) cameras or charge coupled device (CCD) imaging units.

Second embodiment

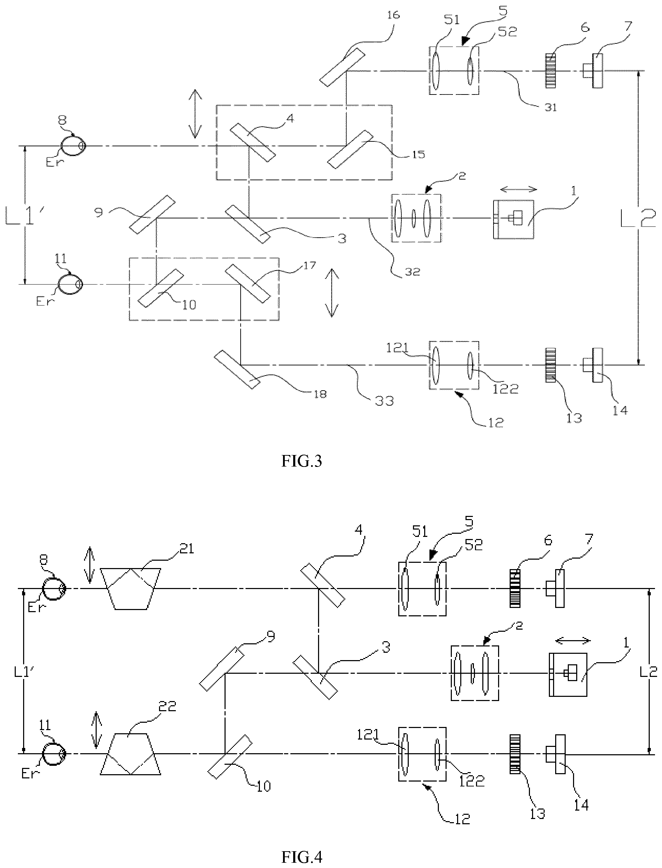

As illustrated in FIG. 3, a beam of light provided by a light source 1 passes through a third lens group 2 composed of several imaging lenses and then is incident to a third beam divider 3, thereafter the beam of light is split into two beams of light. A first beam of light of the two beams of light is reflected by the third beam divider 3 to a first beam divider 4, and a second beam of light of the two beams of light is transmitted by the third beam divider 3 to a first reflector 9. The first beam of light of the two beams of light is reflected by the first beam divider 4 and incident to the fundus oculi Er of a first human eye 8 (e.g., the upper eye), and then scattered by the fundus oculi Er of the first human eye 8. The first beam of light carrying fundus oculi information of the first human eye 8 is returned to the first beam divider 4 and then transmitted by the first beam divider 4 to a second reflector 15, then reflected by the second reflector 15 to a third reflector 16. The beam of light reflected to the third reflector 16 passes through a first lens group 5, and finally images on a first imaging unit 7 after passing through a first micro-lens array 6 to obtain a first dot pattern responsive to light reflected from the fundus oculi (Er) of the first human eye 8 (see Er1 illustrated in FIG. 2). The second beam of light of the two beams of light transmitted by the third beam divider 3 is incident to a first reflector 9 and then reflected by the first reflector 9 to a second beam divider 10. The beam of light received by the second beam divider 10 is reflected to the fundus oculi Er of a second human eye 11 (e.g., the lower eye), and scattered by the fundus oculi Er of the second human eye 11. The second beam of light carrying fundus oculi information of the second human eye 11 is returned to the second beam divider 10 and then transmitted by the second beam divider 10 to a fourth reflector 17, reflected by the fourth reflector 17 to a fifth reflector 18. The beam of light received from the fifth reflector 18 is reflected to a second lens group 12 consisting of several lenses, and finally imaged on a second imaging unit 14 after passing through a second micro-lens array 13 to obtain a second dot pattern responsive to light reflected from the fundus oculi (Er) of the second human eye 11 (see Er2 illustrated in FIG. 2).

Since the light source 1 can be adjusted along the direction of the optical axis of the measuring light to fog the first human eye and the second human eye, for subjects with different diopters, the first dot pattern relating to the fundus oculi Er of the first human eye 8 is clearly formed on the first imaging unit 7 and the second dot pattern relating to the fundus oculi Er of the second human eye 11 is clearly formed on the second imaging unit 14 (see FIG. 2).

Similar with the first embodiment, this binocular refractometer can also measure diopters of two eyes of a subject and whether each of two eyes of the subject have astigmatism and astigmatic axis angle is determined by judging whether figures formed by four dots adjacent in horizontal and vertical directions in the first dot pattern and in the second dot pattern are all squares. The specific measurement method and measurement principle are the same as those of the first embodiment.

At the same time, this binocular refractometer can also measure the pupillary distance L1' of the subject.

As described in the first embodiment, the pupillary distance L1' can be expressed as L1'=L2+K1'+K2'. Therefore, a system pupillary distance L2 after adjusting (a system pupillary distance L2 adjusted), an actual distance K1', and an actual distance K2' are also required. The calculation of K1' and K2' is exactly the same as that in the first embodiment, and will not be elaborated herein. However, calculating the value of the system pupillary distance L2 after adjusting is different from that in the first embodiment. As one implementation, in this embodiment, the second reflector 15 and the first beam divider 4 are adjusted as a whole upward or downward, the second beam divider 10 and the fourth reflector 17 are adjusted as a whole upward or downward, until the system pupillary distance L2 after adjusting meets measuring requirement of the pupillary distance of the subject. As a result, L2 can be greater than, less than, or equal to 65mm. When the system distance L2 is just exactly 65mm, it indicates that this binocular refractometer does not need to make any adjustments.

Therefore, the only difference between the second embodiment and the first embodiment is that optical elements to be adjusted are different during the adjustment of the system distance L2.

Advantages of this embodiment are as follows. After the second reflector 15, the third reflector 16, the fourth reflector 17 and the fifth reflector 18 are added, when measuring the pupillary distances of different subjects, there is no need to move the first beam divider 4, the first lens group 5, the first micro-lens array 6, and the first imaging unit 7 while the four need to be moved in the first embodiment; similarly, there is no need to move the second beam divider 10, the second lens group 12, the second micro-lens array 13, and the second imaging unit 14 as well. It is only necessary to translate the first beam divider 4 and the second reflector 15 as a whole in the direction of the pupillary distance, and translate the second beam divider 10 and the fourth reflector 17 as a whole in the direction of the pupillary distance. It is possible to ensure that an optical axis 31, an optical axis 32, and an optical axis 33 are always parallel to each other, so that the final measured pupillary distance L1' can be more accurate.

Third embodiment

Referring to FIG. 4, in this embodiment, a beam of light provided by a light source 1 passes through a third lens group 2 composed of several imaging lenses and then is incident to a third beam divider 3. A part of the beam of light is reflected by the third beam divider 3 to form reflected light, and another part of the beam of light is transmitted by the third beam divider 3 to form transmitted light. The reflected light formed is reflected by a first beam divider 4 to a first Dove prism 21, incident to the fundus oculi Er of a first human eye 8 (e.g., the upper eye) after refracted by the first Dove prism 21, and scattered by the fundus oculi Er of the first human eye 8. Reflected light carrying fundus oculi information of the first human eye 8 is returned to the first Dove prism 21, returned to the first beam divider 4 after refracted by the first Dove prism 21, and then transmitted by the first beam divider 4 to a first lens group 5, finally imaged on a first imaging unit 7 after passing through a first micro-lens array 6 to obtain a first dot pattern of the light reflected from the fundus oculi Er of the first human eye 8 (see Er1 illustrated in FIG. 2). The transmitted light formed by the third beam divider 3 is incident to a first reflector 9 and then reflected by the first reflector 9 to a second beam divider 10, reflected by the second beam divider 10 to a second Dove prism 22, incident to the fundus oculi Er of a second human eye 11 after refracted by the second Dove prism 22, and scattered by the fundus oculi Er of the second human eye 11. Reflected light carrying fundus oculi information of the second human eye 11 is returned to the second Dove prism 22, incident to the second beam divider 10 after refracted by the second Dove prism 22, and then transmitted by the second beam divider 10 to a second lens group 12, finally imaged on a second imaging unit 14 after passing through a second micro-lens array 13 to obtain a second dot pattern of the light reflected from the fundus oculi Er of the second human eye 11 (see Er2 illustrated in FIG. 2).

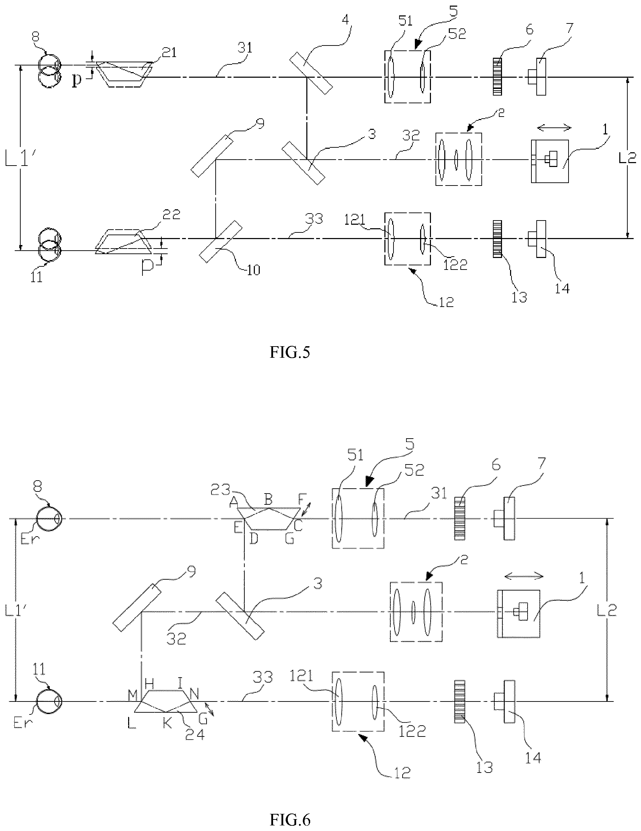

Since pupillary distances of subjects are different, assume that the pupillary distance of a subject is relatively large, in this embodiment (i.e., the third embodiment), it is only necessary to adjust the first Dove prism 21 and the second Dove prism 22 along the direction of the pupillary distance (i.e., an upward direction or a downward direction), see FIG. 5 in detail. Thereafter, a system pupillary distance L2 corresponding to the first human eye 8 and the second human eye 11 will be adjusted accordingly. Assume that the first Dove prism 21 in FIG. 5 is translated upward by a distance p (p is a known value) relative to the first Dove prism 21 in FIG. 4, and the second Dove prism 22 in FIG. 5 is translated downward by a distance P relative to the second Dove prism 22 in FIG. 4, therefore, the system pupillary distance L2 corresponding to the two eyes becomes 65+4p, that is, the value of the system pupillary distance L2 is equal to 65+4p. In general, an initial value of the system pupillary distance L2 is 65mm. Similar with the previous two embodiments, the purpose of adjusting the system pupillary distance L2 is to enable, for different subjects with different pupillary distances, the first human eye 8 to form the first dot pattern of the light reflected from the fundus oculi Er of the first human eye 8 (see FIG. 2) on the first imaging unit 7 and the second human eye 11 to form the second dot pattern of the light reflected from the fundus oculi Er of the second human eye 11 on the second imaging unit 14 (see FIG. 2). It is to be noted that, system pupillary distances of many different subjects are not set to be the initial value 65mm.

In this embodiment, the first Dove prism 21 and the second Dove prism 22 have the same specifications.

As the light source 1 can be adjusted along the direction of the optical axis of the measuring light to fog the first human eye and the second human eye, for subjects with different diopters, a dot pattern relating to the fundus oculi Er of the first human eye 8 can be always clearly formed on the first imaging unit 7 (see FIG. 2), and a dot pattern relating to the fundus oculi Er of the second human eye 11 can be always clearly formed on the second imaging unit 14 (see FIG. 2).

As described in the first embodiment, this binocular refractometer can also measure the diopters of two eyes of the subject and whether each of two eyes of the subject have astigmatism and astigmatic axis angle is determined by judging whether figures formed by four dots adjacent in horizontal and vertical directions in the first dot pattern and in the second dot pattern are all squares. The specific measurement method and measurement principle are the same as those of the first embodiment, and will not be described in further detail herein.

At the same time, this binocular refractometer can also measure the pupillary distance L1' of the subject.

Similarly, in this embodiment, the pupillary distance L1' can be defined as L1'=L2+K1'+K2'=65mm+4P+K1'+K2'. Therefore, the system pupillary distance L2 after adjusting (system pupillary distance L2 adjusted), an actual distance K1', and an actual distance K2' are also required. The calculation of K1' and K2' is exactly the same as that in the first embodiment, and will not be detailed repeatedly herein. However, calculating the value of the system pupillary distance L2 after adjusting is different from that in the first embodiment. As one implementation, in this embodiment, the adjustment of the system pupillary distance L2 is realized by adjusting the first Dove prism 21 and the second Dove prism 22 along the direction of the pupillary distance.

Advantages of this embodiment are as follows. When measuring the pupillary distance, it is only necessary to adjust the first Dove prism 21 and the second Dove prism 22 along the direction of the pupillary distance without any further adjustment on other optical elements. Therefore, the parallel relationship between an optical axis 31, an optical axis 32, and an optical axis 33 can be easily ensured, so that the pupillary distance L1' finally measured can be more accurate. Meanwhile, when the first Dove prism 21 is adjusted along the direction of the pupillary distance, an optical path from the first human eye 8 to the first lens group 5 will not change; when the second Dove prism 22 is adjusted along the direction of the pupillary distance, an optical path from the second human eye 11 to the second lens group 12 will also remain unchanged.

Fourth embodiment

Referring to FIG. 6, a beam of light provided by a light source 1 passes through a third lens group 2 and is incident to a third beam divider 3. A part of the beam of light is reflected by the third beam divider 3, and another part of the beam of light is transmitted by the third beam divider 3. The light reflected by the third beam divider 3 is incident to a point E on an incident surface AD of a third Dove prism 23, reflected by the incident surface AD to the fundus oculi Er of a first human eye 8, and then scattered by the fundus oculi Er of the first human eye 8. Reflected light carrying fundus oculi information of the first human eye 8 is returned to the point E, refracted by the incident surface AD and incident to a point B of a reflective surface AF, reflected at the point B to a point C, and passes through a first lens group 5 after refracted at the point C, finally imaged on a first imaging unit 7 after passing through a first micro-lens array 6 to obtain a first dot pattern of the light reflected from the fundus oculi Er of the first human eye 8 (see Er1 illustrated in FIG. 2). The beam of light transmitted by the third beam divider 3 is incident to a first reflector 9 and then reflected by the first reflector 9 to a point M of an incident surface HL of a fourth Dove prism 24, reflected by the point M to the fundus oculi Er of a second human eye 11, and then scattered by the fundus oculi Er of the second human eye 11. Reflected beam of light carrying fundus oculi information of the second human eye 11 is incident to the point M, refracted at the point M, incident to a point K, and then reflected to a point N, transmitted to a second lens group 12 after refracted at the point N, finally imaged on a second imaging unit 14 after passing through a second micro-lens array 13 to obtain a second dot pattern of the light reflected from the fundus oculi Er of the second human eye 11 (see Er2 illustrated in FIG. 2).

In this embodiment, the third Dove prism 23 and the fourth Dove prism 24 have the same specifications

Since the light source 1 can be adjusted along the direction of the optical axis of the measuring light to fog the first human eye and the second human eye, for subjects with different diopters, the first dot pattern relating to the fundus oculi Er of the first human eye 8 can be always clearly formed on the first imaging unit 7, and the second pattern relating to the fundus oculi Er of the second human eye 11 can be always clearly formed on the second imaging unit 14 (see FIG. 2).

As in the first embodiment, this binocular refractometer can also measure diopters of two eyes of a subject and whether each of two eyes of the subject have astigmatism and astigmatic axis angle is determined by judging whether figures formed by four dots adjacent in horizontal and vertical directions in the first dot pattern and in the second dot pattern are all squares. The specific measurement method and measurement principle are the same as those of the first embodiment, and it will not be repeated herein.

At the same time, this binocular refractometer can also measure the pupillary distance L1' of the subject.

As described in the first embodiment, the pupillary distance L1' can be expressed as L1'=L2+K1'+K2'. Therefore, a system pupillary distance L2 after adjusting (system pupillary distance L2 adjusted), an actual distance K1', and an actual distance K2' are also required. The calculation of K1' and K2' is exactly the same as that in the first embodiment, and will not be elaborated herein. However, the calculation method of the value of the system pupillary distance L2 after adjusting is different from that in the first embodiment. Similarly, as in the previous embodiments, the system pupillary distance L2 in this embodiment needs to be adjusted as well. Referring to FIG. 6, in this embodiment, the third Dove prism 23 is adjusted upward or downward along a FG direction and the fourth Dove prism 24 is adjusted upward or downward along the direction IG, until the first dot pattern relating to the fundus oculi Er of the first human eye 8 is clearly formed on the first imaging unit 7 and the second dot pattern relating to the fundus oculi Er of the second human eye 11 is clearly formed on the second imaging unit 14 (see FIG. 2). Finally, the pupillary distance L1' of the subject can be calculated according to the formula L1'=L2+K1'+K2'.

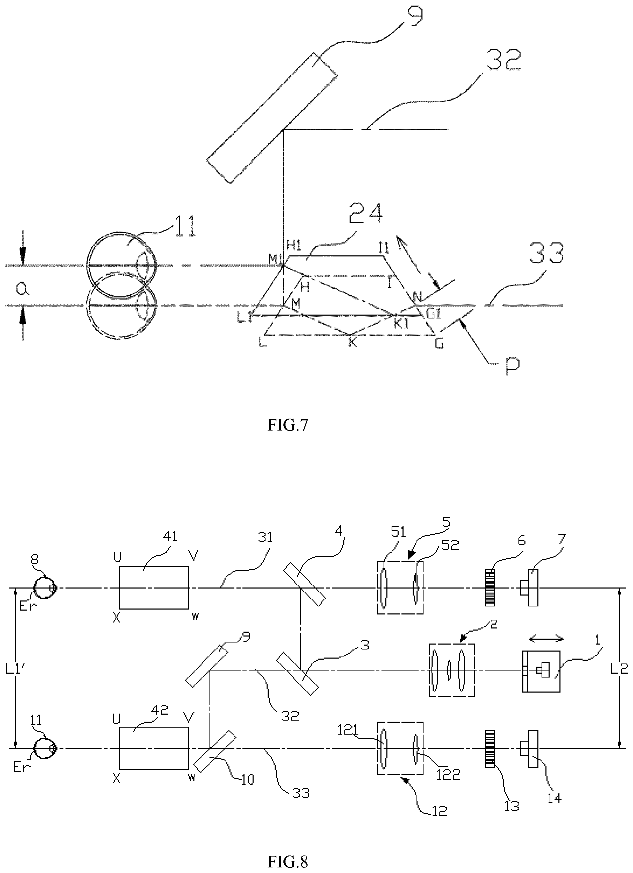

Referring to FIG. 7, the following explains in detail the reason why, when the fourth Dove prism 24 is adjusted along the direction of an emergence surface GI (obliquely upward), the returned optical path can still be provided in parallel from a point N (the same principle applies to the obliquely downward adjustment). Assume that the pupillary distance of a subject is measured and a system pupillary distance suitable for the subject is less than an initial value of the system pupillary distance (65mm), the fourth Dove prism 24 needs to be adjusted obliquely upward and the third Dove prism 23 needs to be adjusted obliquely downward along the direction of an emergence surface FG. When the fourth Dove prism 24 is adjusted obliquely upward, an incident point of the beam of light originally incident by the first reflector 9 to the fourth Dove prism 24 is changed from a point M to a point M1. The beam of light incident at the point M1 is reflected at the point M1 to the fundus oculi Er of the second human eye 11 and then scattered by the fundus oculi Er of the second human eye 11, returned to the point M1, and refracted at the point M1. The light refracted at the point M1 is reflected at a point K1 of a surface L1G1 of the fourth Dove prism 24. The reflected light KIN is also refracted at a point N, the refracted light generated is provided in parallel from the point N. It can be seen that, no matter the fourth Dove prism 24 is translated inward or outward along the direction of the emergence surface GI, the emergence position of the beam of light finally provided from the N point remains unchanged. The same applies to the third Dove prism 23.

Consequently, when adjusting the system pupillary distance L2 of this binocular refractometer, no matter how the third Dove prism 23 and the fourth Dove prism 24 are translated along the corresponding slant surfaces to satisfy the measuring requirements of subjects with different pupillary distances, in the end, the first human eye 8 can still image on the first imaging unit 7 and the second human eye 11 can also image on the second imaging unit 14. Moreover, there is no need to translate the first lens group 5, the first micro-lens array 6, the first imaging unit 7, the second lens group 12, the second micro-lens array 13, and the second imaging unit 14.

When a translation distance of the fourth Dove prism 24 is p, an upward adjustment distance of the second human eye 11 (defined as a) is $2p*\sin45°$. Since a distance that the third Dove prism 23 needs to be adjusted obliquely downward is the same as that of the fourth Dove prism 24, a distance of two eyes are inward adjusted 2a, that is, $4p*\sin45°$. Therefore, the value of the system pupillary distance L2 after adjusting is $65-4p*\sin45°$.

Finally, the real pupillary distance L1' of the subject is determined according to the formula $L1'=L2+K1'+K2'=65-4p*\sin45°+K1'+K2'$. It should be noted that the real pupillary distance can be expressed as $L1'=65+4p*\sin45°+K1'+K2'$ when the fourth Dove prism 24 and the third Dove prism 23 move outward respectively.

In this embodiment, the incident surface AD of the third Dove prism 23 is a transflective surface and the emergence surface FG of the third Dove prism 23 is a totally transparent surface. An angle between the emergence surface FG and a reflective surface AF is 45 degrees. The third Dove prism 23 has a section DGFA that is an isosceles trapezoid. The incident surface HL of the fourth Dove prism 24 is a transflective surface and the emergence surface IG of the fourth Dove prism 24 is a totally transparent surface. An angle between the incident surface HL and a reflective surface LG is 45 degrees. The Dove prism 24 has a section HIGL that is an isosceles trapezoid.

Advantages of this embodiment are as follows. On the one hand, an optical axis 31, an optical axis 32, and an optical axis 33 will not be affected and always be parallel to each other when the third Dove prism 23 and the fourth Dove prism 24 are translated, which is conducive to improving the accuracy of measuring the pupillary distance. On the other hand, compared with the foregoing three embodiments, in this embodiment, the first beam divider 4 and the second beam divider 10 are removed, so that the entire optical path can occupy less space and the layout can be more compact. At the same time, an optical path from the first human eye 8 to the first lens group 5 will not change when the third Dove prism 23 is adjusted along the direction of the pupillary distance and an optical path from the second human eye 11 to the second lens group 12 will also not change when the fourth Dove prism 24 is adjusted along the direction of the pupillary distance.

Fifth embodiment

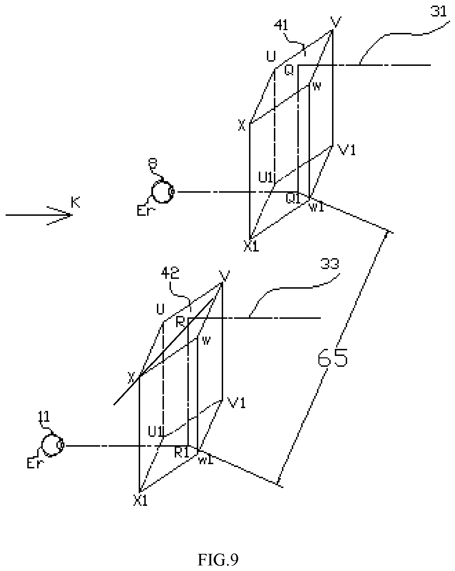

Referring to FIG. 8, a beam of light provided by a light source 1 passes through a third lens group 2 and then is incident to a third beam divider 3. A part of the beam of light is reflected by the third beam divider 3, and another part of the beam of light is transmitted by the third beam divider 3. The beam of light reflected by the third beam divider 3 is incident to a first beam divider 4, reflected by the first beam divider 4 to a slope UVWX of a first prism 41 (see FIG. 9), reflected by the slope UVWX to a slope U1V1W1X1, finally reflected by the slope U1V1W1X1 to a slope UXX1U1 and provided in parallel. The beam of light provided is incident to the fundus oculi Er of a first human eye 8 (e.g., the upper eye), and scattered by the fundus oculi Er of the first human eye 8. Reflected beam of light carrying fundus oculi information of the first human eye 8 is returned by the same way to the first beam divider 4 and then transmitted by the first beam divider 4 to a first lens group 5, finally imaged on a first imaging unit 7 after passing through a first micro-lens array 6 to obtain a first dot pattern of the light reflected from the fundus oculi Er of the first human eye 8 (see Er1 illustrated in FIG. 2). The beam of light transmitted by the third beam divider 3 is incident to a first reflector 9 and then reflected by the first reflector 9 to a second beam divider 10, reflected by the second beam divider 10 to a slope UVWX of a second prism 42 (see FIG. 9), reflected by the slope UVWX to a slope U1V1W1X1, finally reflected by the slope U1V1W1X1 to a slope UXX1U1 and provided in parallel. The beam of light provided is incident to the fundus oculi Er of a second human eye 11 (e.g., the lower eye), and scattered by the fundus oculi Er of the second human eye 11. Reflected beam of light carrying fundus oculi information of the second human eye 11 is returned by the same way to the second beam divider 10 and then transmitted by the second beam divider 10 to a second lens group 12, finally imaged on a second imaging unit 14 after passing through a second micro-lens array 13 to obtain a second dot pattern of the light reflected from the fundus oculi Er of the second human eye 11 (see Er2 illustrated in FIG. 2).

In this embodiment, the first prism 41 and the second prism 42 have the same specifications.

Since the light source 1 can be adjusted along the direction of the optical axis of the measuring light to fog the first human eye and the second human eye, for subjects with different diopters, the first dot pattern relating to the fundus oculi Er of the first human eye 8 can be always clearly formed on the first imaging unit 7, and the second dot pattern relating to the fundus oculi Er of the second human eye 11 can be always clearly formed on the second imaging unit 14 (see FIG. 2).

As in the first embodiment, this binocular refractometer can also measure diopters of two eyes of a subject and whether each of two eyes of the subject have astigmatism and astigmatic axis angle is determined by judging whether figures formed by four dots adjacent in horizontal and vertical directions in the first dot pattern and in the second dot pattern are all squares. The specific measurement method and measurement principle are the same as those of the first embodiment and will not be detailed repeatedly herein.

At the same time, this binocular refractometer can also measure the pupillary distance L1' of the subject.

Similar with the first embodiment, the pupillary distance L1' can be expressed as L1'=L2+K1'+K2'. Therefore, the system pupillary distance L2 after adjusting (system pupillary distance L2 adjusted), an actual distance K1', and an actual distance K2' are also required. The calculation of K1' and K2' is exactly the same as that in the first embodiment, and will not be repeated here.

Since the first prism 41 and the second prism 42 in this embodiment are different from the aforementioned Dove prisms, therefore, in this embodiment, calculating the system pupillary distance L2 after adjusting is different from that in the previous embodiments.

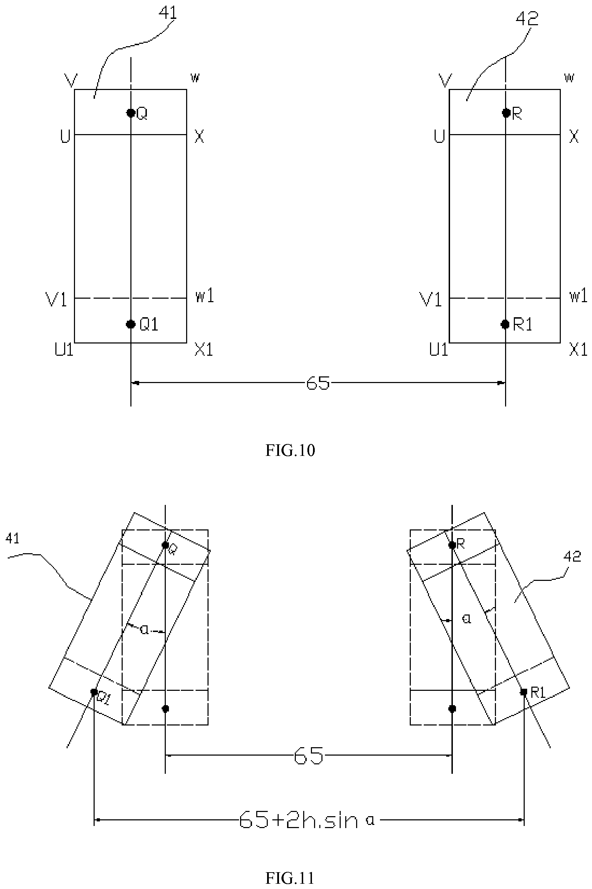

FIG. 10 illustrates a side view of the first prism and the second prism in FIG. 9 when viewed from the direction of the arrow K shown in FIG. 9. A shaft axis of the first prism 41 is QQ1, where Q is a center point of the slope UVWX and Q1 is a center point of the slope U1V1W1X1, and a distance between the center point Q and the center point Q1 is defined as h. A shaft axis of the second prism 42 is RR1, where R is a center point of the slope UVWX and R1 is a center point of the slope U1V1W1X1. Since the first prism 41 and the second prism 42 are exactly the same, a distance between the center point R and the center point R1 is also h. As pupillary distances of different subjects are different, when measuring, the first prism 41 and the second prism 42 need to be adjusted to match measuring needs of different subjects. Elaborate adjusting manner is illustrated in FIG. 11. Assume that the system pupillary distance is 65 mm when the first prism 41 and the second prism 42 are not adjusted (see FIG. 10). For a subject with the pupillary distance that is relatively large, the first prism 41 need to be rotated outward by $\alpha$ degrees and the second prism 42 also need to be rotated outward by $\alpha$ degrees. When rotating, the shaft axis QQ1 of the first prism 41 is rotated outward by an angle $\alpha$ with Q as a rotation center, and the shaft axis RR1 of the second prism 42 is rotated by an angle $\alpha$ with R as a rotation center. Therefore, the system pupillary distance after adjusting can be expressed as $L2=65+2h*\sin\alpha$ and the pupillary distance of the two eyes can be expressed as $L1'=L2+K1'+K2'= 65+2h*\sin\alpha+K1'+K2'$.

In this embodiment, the first prism 41 has slopes UVWX and U1V1W1X1 both forming an angle of 45 degrees with a horizontal plane and the second prism 42 has slopes UVWX and U1V1W1X1 both forming an angle of 45 degrees with the horizontal plane.

Advantages of this embodiment are as follows. It is only necessary to rotate the first prism 41 and the second prism 42 outward or inward at the same time without any further adjustment on other optical elements, and therefore the purpose of ensuring the accuracy of measuring the pupillary distance L1' can be achieved. Meanwhile, the optical axis 31, the optical axis 32, and the optical axis 33 in FIG. 8 will not be affected in positions and remain parallel to each other and as a result, the purpose of ensuring the accuracy of measurement can also be achieved.

In the above five embodiments, the initial value of the system pupillary distance L2 set by this binocular refractometer is always 65mm, and the value of the system pupillary distance L2 is changed after adjusted by the binocular refractometers in the above five embodiments. In this case, the system pupillary distance L2 may be greater or less than 65 mm, and it is also possible that the system distance L2 is equal to 65mm. After L2 is adjusted, the distance K1' at which the axis of the first human eye 8 deviates from the optical axis of the first lens group 5 in the first fundus oculi imaging light path can be obtained, the distance K2' at which the axis of the second human eye 11 deviates from the optical axis of the second lens group 12 in the second fundus oculi imaging light path can also be obtained, and finally the pupillary distance L1' can be calculated according to the formula L1'=L2+K1'+K2'. The pupillary distance L1' can be displayed in real time by a displaying module (not shown) provided in this binocular refractometer. In the aforementioned five embodiments, the principle of measuring the diopters, and astigmatism and axial position are the same, while there is a slight difference in measuring the pupillary distance L1'.

While the present disclosure has been described in detail above with reference to the exemplary embodiments, the scope of the present disclosure is not limited thereto. As will occur to those skilled in the art, the present disclosure is susceptible to various modifications and changes without departing from the spirit and principle of the present disclosure. Therefore, the scope of the present disclosure should be determined by the scope of the claims.

**Claims**

1. A binocular refractometer, comprising:

   a light source (1), configured to provide measuring light; and
   a third lens group (2), configured to converge the measuring light provided by the light source (1);
   wherein the binocular refractometer further comprises:
   a light splitting apparatus, configured to split the measuring light provided by the light source (1) and converged by the third lens group (2) into two beams of light, wherein

   a first beam of light of the two beams of light passes through a first fundus oculi imaging light path and illuminates a first human eye (8), and the returned first beam of light carrying fundus oculi information of the first human eye (8) forms, on a first imaging unit (7) in the first fundus oculi imaging light path, a first dot pattern responsive to light reflected from the fundus oculi (Er) of the first human eye (8); and
   a second beam of light of the two beams of light passes through a second fundus oculi imaging light path and illuminates a second human eye (11), and the returned second beam of light carrying fundus oculi information of the second human eye (11) forms, on a second imaging unit (14) in the second fundus oculi imaging light path, a second dot pattern responsive to light reflected from the fundus oculi (Er) of the second human eye (11); and
   an optical element adjusting apparatus, configured to:

   adjust optical elements in the first fundus oculi imaging light path and optical elements in the second fundus oculi imaging light path along the direction of the pupillary distance; and
   adjust the light source (1) along the direction of the optical axis of the measuring light to fog the first human eye (8) and the second human eye (11), whereby, for subjects with different diopters, the first dot pattern relating to the fundus oculi of the first human eye (8) is clearly formed on the first imaging unit (7) and the second dot pattern relating to the fundus oculi of the second human eye (11) is clearly formed on the second imaging unit (14); wherein the light source (1) is conjugated to the fundus oculi (Er) of the first human eye (8) predetermined by the third lens group (2) and the light source (1) is conjugated to the fundus oculi (Er) of the second human eye (11) predetermined by the third lens group (2).

2. The binocular refractometer of claim 1, wherein
   the first fundus oculi imaging light path comprises a first beam divider (4), a first lens group (5), a first micro-lens array (6), and the first imaging unit (7) sequentially arranged along the first fundus oculi imaging light path;
   the second fundus oculi imaging light path comprises a first reflector (9), a second beam divider (10), a second lens group (12), a second micro-lens array (13), and the second imaging unit (14) sequentially arranged along the second fundus oculi imaging light path;
   the light splitting apparatus is a third beam divider (3);
   the first dot pattern is formed on the first imaging unit (7), by converging the first beam of light carrying the fundus oculi information of the first human eye (8) through the first micro-lens array (6); and
   the second dot pattern is formed on the second imaging unit (14), by converging the second beam of light carrying the fundus oculi information of the second human eye (11) through the second micro-lens array (13).

3. The binocular refractometer of claim 2, wherein
the first fundus oculi imaging light path further comprises a second reflector (15) and a third reflector (16) that are disposed between the first beam divider (4) and the first lens group (5); and
the second fundus oculi imaging light path further comprises a fourth reflector (17) and a fifth reflector (18) that are disposed between the second beam divider (10) and the second lens group (12).

4. The binocular refractometer of claim 2, wherein
the first fundus oculi imaging light path further comprises a first Dove prism (21) disposed between the first human eye (8) and the first beam divider (4);
the second fundus oculi imaging light path further comprises a second Dove prism (22) disposed between the second human eye (11) and the second beam divider (10); and
the first Dove prism (21) and the second Dove prism (22) have the same specifications.

5. The binocular refractometer of claim 2, wherein
the first fundus oculi imaging light path further comprises a first prism (41) disposed between the first human eye (8) and the first beam divider (4);
the second fundus oculi imaging light path further comprises a second prism (42) disposed between the second human eye (11) and the second beam divider (10); and
the first prism (41) and the second prism (42) have the same specifications.

6. The binocular refractometer of claim 5, wherein the first prism (41) has slopes (UVWX and U1V1W1X1) both forming an angle of 45 degrees with a horizontal plane and the second prism (42) has slopes (UVWX and U1V1W1X1) both forming an angle of 45 degrees with the horizontal plane.

7. The binocular refractometer of claim 1, wherein
the first fundus oculi imaging light path comprises a third Dove prism (23), a first lens group (5), a first micro-lens array (6), and the first imaging unit (7) sequentially arranged along the first fundus oculi imaging light path;
the second fundus oculi imaging light path comprises a first reflector (9), a fourth Dove prism (24), a second lens group (12), a second micro-lens array (13), and the second imaging unit (14) sequentially arranged along the second fundus oculi imaging light path;
the third Dove prism (23) and the fourth Dove prism (24) have the same specifications;
the light splitting apparatus is a third beam divider (3);
the first dot pattern is formed on the first imaging unit (7), by converging the first beam of light carrying the fundus oculi information of the first human eye (8) through the first micro-lens array (6); and
the second dot pattern is formed on the second imaging unit (14), by converging the second beam of light carrying the fundus oculi information of the second human eye (11) through the second micro-lens array (13).

8. The binocular refractometer of claim 7, wherein
the third Dove prism (23) has an incident surface (AD) that is a transflective surface and an emergence surface (FG) that is a totally transparent surface, an angle between the emergence surface (FG) and a reflective surface (AF) is 45 degrees, and the third Dove prism (23) has a section (DGFA) that is an isosceles trapezoid; and
the fourth Dove prism (24) has an incident surface (HL) that is a transflective surface and an emergence surface (IG) that is a totally transparent surface, an angle between the incident surface (HL) and a reflective surface (LG) is 45 degrees, and the fourth Dove prism (24) has a section (HIGL) that is an isosceles trapezoid.

9. The binocular refractometer according to any one of claims 1-8, wherein the first imaging unit (7) and the second imaging unit (14) are both complementary metal oxide semiconductor (CMOS) cameras or charge coupled device (CCD) imaging units.

10. A method for measuring astigmatism and astigmatic axis angle of two eyes by the binocular refractometer according to any one of claims 1-9, comprising the following operations:

S1, splitting, by a third beam divider (3), measuring light into two beams of light after the measuring light provided by a light source (1) is converged by a third lens group (2), wherein

a first beam of light of the two beams of light passes through a first fundus oculi imaging light path and illuminates a first human eye (8), and the returned first beam of light carrying fundus oculi information of the first human eye (8) forms, on a first imaging unit (7) in the first fundus oculi imaging light path, a first dot pattern responsive to light reflected from the fundus oculi (Er) of the first human eye (8); and

a second beam of light of the two beams of light passes through a second fundus oculi imaging light path and illuminates a second human eye (11), and the returned second beam of light carrying fundus oculi information of the second human eye (11) forms, on a second imaging unit (14) in the second fundus oculi imaging light path, a second dot pattern responsive to light reflected from the fundus oculi (Er) of the second human eye (11);

S2, adjusting, by an optical element adjusting apparatus, the first fundus oculi imaging light path and the second fundus oculi imaging light path in the direction of the pupillary distance, and adjusting the light source (1) along the direction of the optical axis of the measuring light to fog the first human eye (8) and the second human eye (11), whereby, for subjects with different diopters, the first dot pattern relating to the fundus oculi of the first human eye (8) is clearly formed on the first imaging unit (7) and the second dot pattern relating to the fundus oculi of the second human eye (11) is clearly formed on the second imaging unit (14); and

S3, indicating that two eyes of the subject has no astigmatism and no astigmatic axis angle based on a determination that figures formed by four dots adjacent in horizontal and vertical directions in each of the first dot pattern displayed on the first imaging unit (7) and the second dot pattern displayed on the second imaging unit (14) are all squares, or indicating that two eyes of the subject has the astigmatism and the astigmatic axis angle based on a determination that figures formed by four dots adjacent in the horizontal and vertical directions in each of the first dot pattern displayed on the first imaging unit (7) and the second dot pattern displayed on the second imaging unit (14) are all rectangles or parallelograms.

11. A method for measuring diopters of two eyes by the binocular refractometer according to any one of claims 1-9, comprising the following operations:

S1, splitting, by a third beam divider (3), measuring light into two beams of light after the measuring light provided by a light source (1) is converged by a third lens group (2), wherein

a first beam of light of the two beams of light passes through a first fundus oculi imaging light path and illuminates a first human eye (8), and the returned first beam of light carrying fundus oculi information of the first human eye (8) forms, on a first imaging unit (7) in the first fundus oculi imaging light path, a first dot pattern responsive to light reflected from the fundus oculi (Er) of the first human eye (8); and

a second beam of light of the two beams of light passes through a second fundus oculi imaging light path and illuminates a second human eye (11), and the returned second beam of light carrying fundus oculi information of the second human eye (11) forms, on a second imaging unit (14) in the second fundus oculi imaging light path, a second dot pattern responsive to light reflected from the fundus oculi (Er) of the second human eye (11);

S2, adjusting, by an optical element adjusting apparatus, the first fundus oculi imaging light path and the second fundus oculi imaging light path in the direction of the pupillary distance, and adjusting the light source (1) along the direction of the optical axis of the measuring light to fog the first human eye (8) and the second human eye (11), whereby, for subjects with different diopters, the first dot pattern relating to the fundus oculi of the first human eye (8) is clearly formed on the first imaging unit (7) and the second dot pattern relating to the fundus oculi of the second human eye (11) is clearly formed on the second imaging unit (14); and

S3, calculating a diopter (D) of the first human eye (8), according to a distance between two adjacent dots in the first dot pattern on the first imaging unit (7) and parameters set by the binocular refractometer, and calculating a diopter (D) of the second human eye (11), according to a distance between two adjacent dots in the second dot pattern on the second imaging unit (14) and the parameters set by the binocular refractometer.

12. A method for measuring the pupillary distance by the binocular refractometer according to any one of claims 1-9, comprising the following operations:

S1, measuring diopters D of two eyes of a subject, by the method according to claim 11;

S2, measuring a distance K1 at which an image (Er1) deviates from a center O1 of the first imaging unit (7), wherein the image (Er1) is formed on a first imaging unit (7) by light reflected from the fundus oculi (Er) of a first human eye (8), measuring a distance K2 at which an image (Er2) deviates from a center O2 of the second imaging unit (14), wherein the image (Er2) is formed on a second imaging unit (14) by light reflected from the fundus oculi (Er) of a second human eye (11), and measuring a system pupillary distance (L2) adjusted;

S3, substituting K1 and K2 measured at S2 for K in a formula respectively:

$$K' = \frac{K\left(\dfrac{m \cdot f \cdot D + 1000 f}{(m-f)\cdot D + 1000} - \dfrac{m \cdot f}{m+f}\right)}{\left(\dfrac{m \cdot f \cdot D + 1000 f}{(m-f)D + 1000} - d\right)} * \frac{m+f}{f}$$

,

calculating a distance K1' at which the axis of the first human eye (8) deviates from the optical axis of a first lens group (5) in a first fundus oculi imaging light path and a distance K2' at which the axis of the second human eye (11) deviates from the optical axis of a second lens group (12) in a second fundus oculi imaging light path, wherein, in the formula,

d is a distance, in a measuring light path, between a rear principal plane of the first lens group (5) and the first imaging unit (7);

f is a focal length of the first lens group (5) in the measuring light path;

D is the measured diopter of the measured eye of the subject; and

m is a distance between a pupil and a front principal plane of the first lens group (5); and

S4, substituting L2 obtained at S2 as well as K1' and K2' obtained at S3 into a formula L1'= L2+K1'+K2', to obtain the pupillary distance (L1') of the subject.

FIG.1

FIG.2

FIG.2-1

FIG.2-2

FIG.3

FIG.4

FIG.5

FIG.6

FIG.7

FIG.8

FIG.9

FIG.10

FIG.11

## INTERNATIONAL SEARCH REPORT

| International application No. |
| --- |
| **PCT/CN2016/075792** |

### A. CLASSIFICATION OF SUBJECT MATTER

A61B 3/103 (2006.01) i

According to International Patent Classification (IPC) or to both national classification and IPC

### B. FIELDS SEARCHED

Minimum documentation searched (classification system followed by classification symbols)

A61B 3/-

Documentation searched other than minimum documentation to the extent that such documents are included in the fields searched

Electronic data base consulted during the international search (name of data base and, where practicable, search terms used)

CNPAT, WPI, EPODOC, CNKI, ResearchGate: SHENZHEN MOPTIM IMAGING TECHNIQUE CO., LTD.; GUO, Shuguang; diopter, interpupillary distance, optometry, dot matrix, astigmatism, scieropia, lenticule, facula, binocular, half mirror, fogging, square, refract+, astigmatism, Hartmann, wavefront, wave-front, microoptics, dual, binocular, spot

### C. DOCUMENTS CONSIDERED TO BE RELEVANT

| Category* | Citation of document, with indication, where appropriate, of the relevant passages | Relevant to claim No. |
| --- | --- | --- |
| X | HAMPSON, K.M. et al. "Binocular Shack-Hartmann Sensor for the Human Eye.", JOURNAL OF MODERN OPTICS., vol. 55, no. 4, 01 February 2008 (01.02.2008), ISSN: 1362-3044, pages 703-716 | 1-9 |
| A | US 2012038884 A1 (FERNANDEZ, M.; ENRIQUE, J. et al.), 16 February 2012 (16.02.2012), the whole document | 1-9 |
| A | US 2002097376 A1 (APPLEGATE, R.A. et al.), 25 July 2002 (25.07.2002), the whole document | 1-9 |
| A | US 6382795 B1 (CARL ZEISS, INC.), 07 May 2002 (07.05.2002), the whole document | 1-9 |
| A | CN 104257346 A (JILIN UNIVERSITY), 07 January 2015 (07.01.2015), the whole document | 1-9 |
| A | US 2006126019 A1 (LIANG, Junzhong et al.), 15 June 2006 (15.06.2006), the whole document | 1-9 |

☐ Further documents are listed in the continuation of Box C.          ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 31 March 2016 (31.03.2016) | **01 June 2016 (01.06.2016)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **SONG, Han** Telephone No.: (86-10) **62413517** |
| --- | --- |

Form PCT/ISA/210 (second sheet) (July 2009)

**INTERNATIONAL SEARCH REPORT**

International application No.

**PCT/CN2016/075792**

**Box No. II    Observations where certain claims were found unsearchable (Continuation of item 2 of first sheet)**

This international search report has not been established in respect of certain claims under Article 17(2)(a) for the following reasons:

1. ☒    Claims Nos.: 10-12
    because they relate to subject matter not required to be searched by this Authority, namely:
    [1] Claim 10 sets forth a method for detecting binocular astigmatism and axis using said binocular optometry device in any one of claims 1-9. This method judges whether the examinee's binoculus have an astigmatism and axis and is a diagnostic method for treatment of the living human body, which falls within the subject matter that does not warrant a search by the International Searching Authority as defined in PCT Rule 39.1.
    [2] Claim 11 sets forth a method for detecting binocular dioptre using said binocular optometry device in any one of claims 1-9. This method judges whether the binoculus diopter is normal by calculating the examinee's binoculus dioptre and is a diagnostic method for treatment of the living human body, which falls within the subject matter that does not warrant a search by the International Searching Authority as defined in PCT Rule 39.1.
    [3] Claim 12 sets forth a method for detecting binocular pupil distance using said binocular optometry device in any one of claims 1-9. This method includes steps of detecting examinee's binoculus diopter D according to said method for detecting binoculus diopter in claim 11, thus judges whether the binoculus diopter is normal and is a diagnostic method for treatment of the living human body, which falls within the subject matter that does not warrant a search by the International Searching Authority as defined in PCT Rule 39.1.

2. ☐    Claims Nos.:
    because they relate to parts of the international application that do not comply with the prescribed requirements to such an extent that no meaningful international search can be carried out, specifically:

3. ☐    Claims Nos.:
    because they are dependent claims and are not drafted in accordance with the second and third sentences of Rule 6.4(a).

Form PCT/ISA/210 (continuation of first sheet (2)) (July 2009)

# INTERNATIONAL SEARCH REPORT
## Information on patent family members

International application No.

**PCT/CN2016/075792**

| Patent Documents referred in the Report | Publication Date | Patent Family | Publication Date |
|---|---|---|---|
| US 2012038884 A1 | 16 February 2012 | ES 2346392 A1 | 14 October 2010 |
| | | WO 2010100298 A1 | 10 September 2010 |
| | | ES 2346392 B1 | 03 October 2011 |
| | | EP 2404546 A1 | 11 January 2012 |
| | | US 8506079 B2 | 13 August 2013 |
| US 2002097376 A1 | 25 July 2002 | US 6659613 B2 | 09 December 2003 |
| | | US 2004119942 A1 | 24 June 2004 |
| | | US 6905210 B2 | 14 June 2005 |
| US 6382795 B1 | 07 May 2002 | DE 60121123 D1 | 10 August 2006 |
| | | EP 1217939 B1 | 28 June 2006 |
| | | DE 60121123 T2 | 01 February 2007 |
| | | EP 1217939 A2 | 03 July 2002 |
| | | JP 2003533320 A | 11 November 2003 |
| | | WO 0189372 A2 | 29 November 2001 |
| CN 104257346 A | 07 January 2015 | None | |
| US 2006126019 A1 | 15 June 2006 | WO 2007064363 A1 | 07 June 2007 |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5777718 A
- CN 101718542 B
- WO 2005048829 A2
- CN 102988022 A